Europäisches Patentamt

European Patent Office

Office européen des brevets

(11) Publication number: **0 103 036**
**A1**

## EUROPEAN PATENT APPLICATION

(21) Application number: 82108328.4

(22) Date of filing: 09.09.82

(51) Int. Cl.³: **A 61 M 5/14,** F 16 K 7/06

(43) Date of publication of application: 21.03.84
**Bulletin 84/12**

(84) Designated Contracting States: **AT BE CH DE FR GB IT LI LU NL SE**

(71) Applicant: **Ueda, Shiro, 156, Fu Hsing North Road, Taipei (TW)**

(72) Inventor: **Ueda, Shiro, 156, Fu Hsing North Road, Taipei (TW)**

(74) Representative: **Casalonga, Axel et al, BUREAU D.A. CASALONGA OFFICE JOSSE & PETIT Baaderstrasse 12-14, D-8000 München 5 (DE)**

(54) Clamp regulator for transfusing liquid and blood.

(57) Clamp regulator for transfusing liquid and blood, especially formed by an adjustable wheel (6) and an auxiliary wheel (7). The flow rate inside the pipe (5) mounted in the groove of the adjustable wheel (6) can be regulated by the adjustable wheel (6), which not only can be slightly regulated, but also can be maintained and can not be shifted by shocking or with time.

EP 0 103 036 A1

1

Clamp regulator for transfusing liquid and blood.

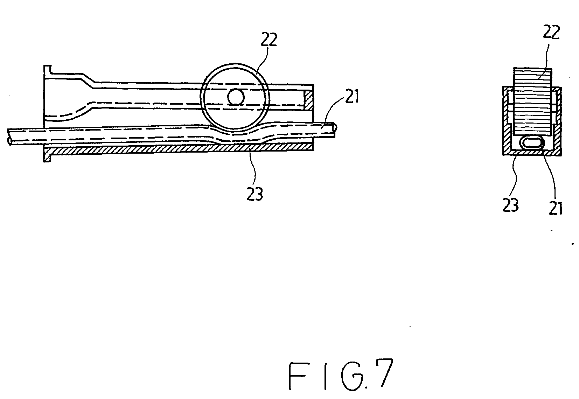

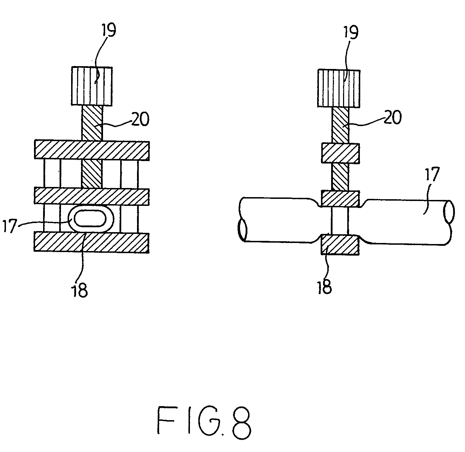

This new invention relates to a clamp regulator for transfusing liquid and blood. The clamps or prior regulators make use of rolling or screwing. The rolling method (please refer to Fig. 7) is that a pipe (21) is inserted between the inclined surface (23) of the bottom and a round rod (22). The round rod (22) is translated by rotation so that the flow rate can be regulated by tightening or enlarging the clearance of the pipe (21). If the round rod excessively tends to be an ellipse or a plane, the flow rate can not be regulated. Furthermore, the flow rate is rapidly decreased and difficult to be slightly regulated. The screwing method (please refer to Fig. 8) is that a pipe (17) is inserted in the bottom of the plane (or in a round shape) and an adjustable rod (19) having screw thread (20) on it allows the adjustable rod (19) to rotate upward and downward, so that the flow rate can be adjusted with the press-plate at the upper part of the bottom (18) and the pipe (17) by pressing the pipe (17). This kind of method, in comparison with the rolling method, permits a more accurate regulation. Usually, the flow rate is regulated to the required value when liquid or blood is transfused. Then injection begins with a hand grasping a clamp and another hand a stop watch. Nevertheless, the inconvenience to rotate the adjustable rod (19) with a hand and fix the bottom (18) with another while regulating in accordance with the screwing method is a drawback.

Regardless of rolling method or screwing method, there is a common drawback : during injection, the flow rate inside the pipes (21), (17) will be gradually decreased with time due to the gradual decrease of the force tending to recover the round shape of the pipes (21), (17), the reaction force and the elastic force of material, causes clearance in the left and the right spaces, widening of the pipes and sinking at the central parts of the pipes (21), (17), so that the area passed by the flow diminishes. Even the clamping of screwing method has the same problem. When the other liquid is injected during liquid transfusion or blood transfusion of mingled injection, the pipes (21),(17) will be oscillated on account of the adjusting portion being small and the clamps being mounted to the central parts of the pipes (21),

2

(17).

The present invention relates to a clamp regulator for transfusing liquid and blood, especially comprising an assembly formed by an adjustable wheel and an auxiliary wheel. The flow rate inside the pipe mounted in the groove of the adjustable wheel can be regulated by the adjustable wheel, which not only can be slightly regulated but also can be maintained and can not be shifted by shocking or with time.

The main purpose of the present invention is to offer a clamp regulator able to be simply adjusted for transfusing liquid and blood.

The second purpose of the present invention is to offer a clamp regulator for transfusing liquid and blood to adjust the flow rate inside the pipe no matter how great the shocking is or how long the time has elapsed, the initial flow rate inside the pipe being maintained.

Fig. 1 is a perspective view of the present invention ;

Fig. 2 is a full-sectional top view of the present invention ;

Fig. 3 is sectional view taken along line II-II of Fig. 2 ;

Fig. 4 is a view in which the adjustable wheel is in different positions ;

Fig. 5 is composed of three sectional views taken along lines III-III, IV-IV, V-V of Fig. 4 respectively ;

Fig. 6 is composed of two side views of different adjustable wheels ;

Fig. 7 is composed of two views of conventional roll regulator ; and

Fig. 8 is composed of two view of conventional screw regulator.

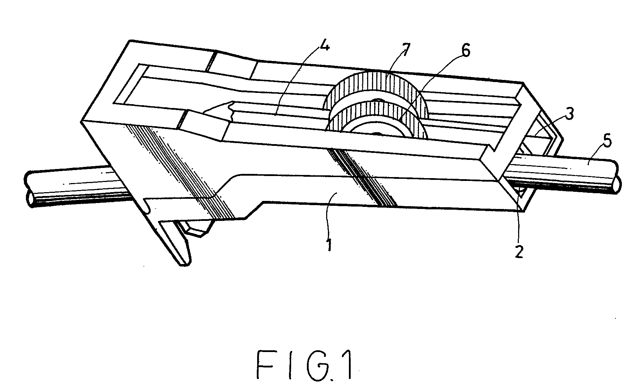

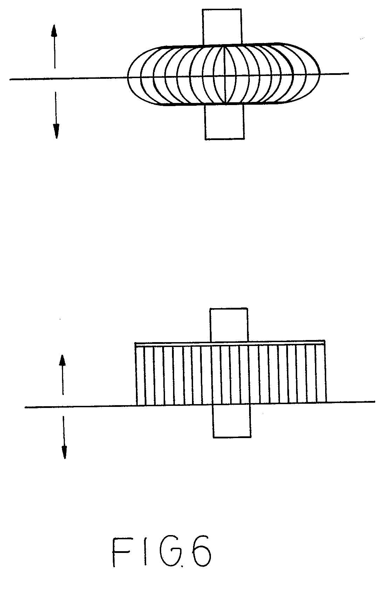

Please refer to Fig. 1 a groove (2) of the adjustable wheel (6) and a groove (3) of the auxiliary wheel (7) are provided in a clamp frame (1), and are divided by a partition (4). The surfaces of the adjustable wheel (6) and the auxiliary wheel (7) are flat. (please refer to Fig. 6) if the surface is circular, clearance will be produced in the central part of the surface of the adjustable wheel ; therefore

3

a flat surface is used to permit for adjustment.

The method of adjusting the flow rate inside the pipe (5) of the clamp frame (1) makes use of the clearance between the inclined plane (9) of the bottom and the adjustable wheel (6) to decrease it, or increase it for effective flow rate adjustment. The characteristics of the present invention are that the width of the groove (2) of the adjustable wheel (6) equals to the outside diameter of pipe (5), and the width of the adjustable wheel (6) is equal to the value obtained by subtracting four times the thickness of pipe (5) from the outside diameter of pipe (5). (Let the width of the adjustable wheel be A, outside diameter of pipe (5) be B, thickness of wall of pipe (5) be C ; therefore, the width of the adjustable wheel (6) is A=B-4C).

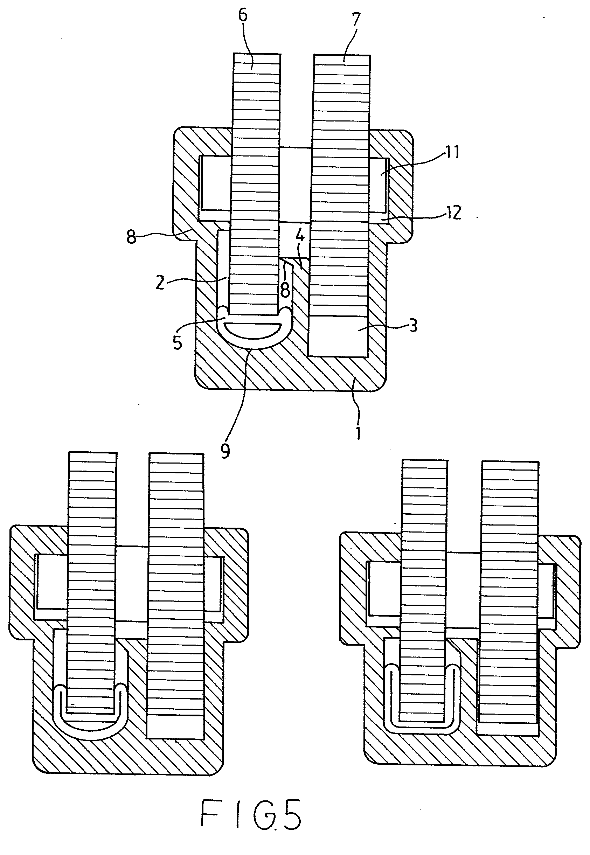

Please refer to Fig. 5 the adjustable wheel (6) and the auxiliary wheel (7) are connnected by an axle (11) which slides in the groove (12), a guided protuberance (8) is provided in groove (2) in the lower part of the adjustable wheel (6) in order to prevent the undulation of the adjustable wheel (6), the groove (3) at the lower part of the auxiliary wheel (7) is tightly engaged with the auxiliary wheel (7) and also has the function of preventing the undulation of the adjustable wheel (6). A space remains between the adjustable wheel (6) and the groove (2) of the adjustable wheel (6) when the pipe (5) is forged into by tight clamping.

It is sure that the present invention can be exactly adjusted and can prevent shocking during transfusing liquid or blood ; therefore, it can be used for accurate transfusion. By reason of the large width of the wheels, it is convenient and accurate.

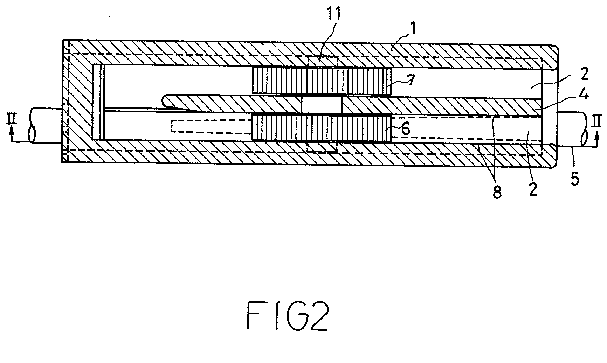

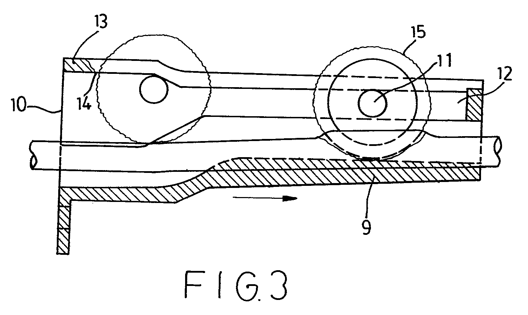

Please refer to Fig. 3. Fig. 3 the sectional view taken from lines II-II of Fig. 2. The length of the inlet (10) of the wheel and the outside diameter of the adjustable wheel (6) are well-matched. Axle (11) is placed into the groove (12) of the clamp frame (1). In the central space which is between the adjustable wheel (6) and the auxiliary wheel (7) is a partition (4) where the wheel is pressed forward to.

The pipe (5) is inserted in the clamp frame (1). When the device is assembled and full flow rate is needed, it is

4

necessary to move the wheel to the return-preventing plate (13) which is opposite ot the arrow direction, and loosen the pipe (5) from the adjusting position of clamp frame (1) ; therefore, full flow rate is obtained. If the wheel is rotated too violently, its indentation (15) in the surface of the wheel will be scraped and engaged by the knife edge (14) at the tip of return-preventing plate (13). If the wheel is lifted a little bit, pipe (5) is easier to be moved. The indentation (15) of the wheel can prevent the slipping of the wheel.

In order to preventary change of the shape of the adjusting position of the pipe (5) in the inclined surface (9) of the bottom of the clamp frame (1) owing ot the move of the wheel, the inclined surface (9) of the bottom is shaped into a conus. No matter how long the time has elapsed, the adjusting position remains unchanged.

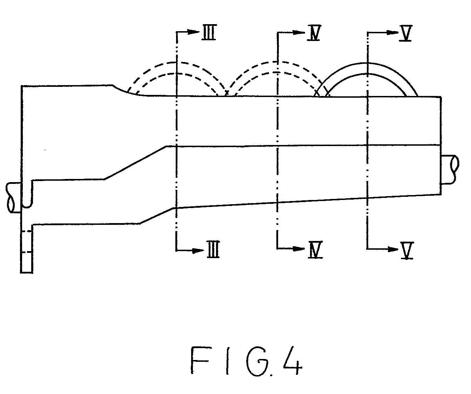

Please refer to Fig. 4 and Fig. 5. The change of the adjusted shape of the pipe (5) and the change of the shape of the inclined surface (9) of the bottom are shown in Fig. 5 which is composed of three sectional views in which the wheels are in different positions due to the rotary translation of the wheels while adjusting the flow rate. It is the above description that the inclined surface (9) of the bottom is shaped into a cone.

When the space between the adjustable wheel (6) and the inclined surface (9) of the bottom is diminished, the shape of the pipe (5) changes from roundness to ellipse by reason of the pressure of the adjustable wheel (6). In addition, the width of the adjustable wheel (6) is equal to the outside diameter of the pipe (5) and the pipe (5) is pressed, so that the rim of the pipe (5) is forced into the clearance and gradually inserted into the two inner-side space. The inside cross-sectional area is gradually decreased, so that the flow rate is naturally decreased.

In short, the flow rate inside the pipe (5) can be slighted adjusted and decreased exactly by pressing the wheel so as to let the part where the outside diameter of the pipe (5) is widened with force into the clearance. No matter how great the shocking is, the flow rate can be adjusted accurately, and the wheel will not be loosened due to the fact that the adjusting part is clamped in both sides. Also, the wheel being used for pressing can prevent the sinking of the portion around the adjusting part of the pipe (5).

From the description above, the time can be measured and the regulator can be adjusted to obtain the required value, besides the flow rate initially adjusted will be unchanged with time ; so it can be sure that the liquid or blood are able to be transfused steadily. The characteristics are completely shown during transfusing small amount of liquid.

6

Claim :

1. A clamp regulator for transfusing liquid and blood, comprising an adjustable wheel (6) and an auxiliary wheel (7) mounted in a clamp frame (1), the adjustable wheel and the auxiliary wheel being two whole bodies, which is characterized in that the pipe (5) used for transfusing liquid and blood is placed in the groove (2) of the adjustable wheel (6), the outer diameter of the pipe being equal to the width of the groove (2) of the adjustable wheel (6), the groove (2) of the adjustable wheel and the groove (3) of the auxiliary wheel being engaged with the adjustable wheel and auxiliary wheel respectively which are able to move freely therein the width of the adjustable wheel (6) being equal to the value obtained by subtracting four times the thickness of the pipe from the outer diameter of said pipe, the inclined surface of the bottom (9) of the groove (2) of the adjustable wheel (6) being conical.

FIG.1

2/8

FIG2

FIG.3

FIG.4



FIG.5

FIG.6

0103036

FIG.7

FIG.8

0103036

European Patent Office

**EUROPEAN SEARCH REPORT**

Application number

EP 82 10 8328

## DOCUMENTS CONSIDERED TO BE RELEVANT

| Category | Citation of document with indication, where appropriate, of relevant passages | Relevant to claim | CLASSIFICATION OF THE APPLICATION (Int. Cl. ³) |
|---|---|---|---|
| A | GB-A-2 020 785 (ABBOTT)<br>* Column 5, lines 18-64; figure 5A * | 1 | A 61 M 5/14<br>F 16 K 7/06 |
| A | DE-A-1 934 337 (BAXTER)<br>* Description; figures * | 1 | |
| A | US-A-3 685 787 (ADELBERG)<br>* Column 4, lines 33-40; figures 4,6 * | 1 | |

TECHNICAL FIELDS
SEARCHED (Int. Cl. ³)

A 61 M
F 16 K

The present search report has been drawn up for all claims

| Place of search | Date of completion of the search | Examiner |
|---|---|---|
| THE HAGUE | 31-05-1983 | VANRUNXT J.M.A. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82